# EUROPEAN PATENT SPECIFICATION

(11) **EP 2 073 791 B1**
(45) Date of publication and mention of the grant of the patent: **22.12.2010**
(21) Application number: 07820581.2
(22) Date of filing: 26.09.2007
(51) Int. Cl.: A61K 9/00, A61K 9/28

(54) **CAPECITABINE PEDIATRIC TABLETS**
CAPECITABINTABLETTEN FÜR KINDER
COMPRIMÉS PÉDIATRIQUES DE CAPÉCITABINE

(30) Priority: 06.10.2006 US 850174 P; 24.07.2007 US 951557 P
(43) Date of publication of application: 01.07.2009
(73) Proprietor: F. Hoffmann-La Roche AG, 4070 Basel (CH)
(72) Inventor: BACHYNSKY, Maria Oksana, Nutley, New Jersey 07110 (US); INFELD, Martin Howard, Upper Montclair, New Jersey 07043 (US); RASHED, Mohammad, Randolph, New Jersey 07869 (US); SHAH, Navnit Hargovindas, Clifton, New Jersey 07012 (US)
(74) Representative: Klein, Thomas
(86) International application number: PCT/EP2007/060186
(87) International publication number: WO 2008/040665

(56) References cited:
- EP-A- 0 273 005
- WO-A-99/44580
- WO-A-2006/110800
- WALKO C M ET AL: "Capecitabine: A review" CLINICAL THERAPEUTICS, EXCERPTA MEDICA, PRINCETON, NJ, US, vol. 27, no. 1, January 2005 (2005-01), pages 23-44, XP004778946 ISSN: 0149-2918
- MARSE H ET AL: "Management of adverse events and other practical considerations in patients receiving capecitabine (Xeloda<(>R))" EUROPEAN JOURNAL OF ONCOLOGY NURSING, CHURCHILL LIVINGSTONE, vol. 8, 2004, pages S16-S30, XP004677075 ISSN: 1462-3889

## Description

The present invention relates to a novel rapidly disintegrating pharmaceutical dosage form having as an active ingredient 5'-deoxy-5-fluoro-N-[(pentyloxy)-carbonyl]-cytidine (capecitabine). The new dosage form is suitable for any patient and especially for patients who have difficulty swallowing solid oral dosage forms, including the pediatric and geriatric populations.

Capecitabine is a fluoropyrimidine carbamate with antineoplastic activity. It is an orally administered systemic prodrug of 5'-deoxy-5-fluorouridine (5'-DFUR), an antineoplastic agent. Capecitabine is marketed in the United States by Roche Laboratories under the brand name Xeloda®. The chemical name for capecitabine is 5'-deoxy-5-fluoro-N-[(pentyloxy)-carbonyl]-cytidine and has the following structural formula:

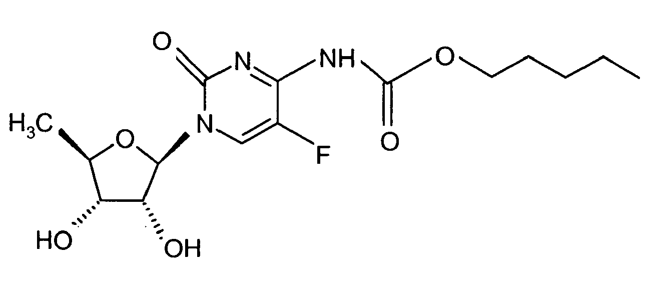

Capecitabine is covered in US patents, including US Pat. No. 4,966,891 and 5,472,949 and USSN 60/667,509, filed April 1, 2005. Improved methods for the manufacture of capecitabine are taught in US Pat. No. 5,453,497 and 5,476,932, and application USSN 60/532,266, filed December 22, 2003. To the extent necessary, any and all of the foregoing patents and applications are herein incorporated by reference.

In the United States, Capecitabine is currently approved for the treatment of colon and breast cancer. The currently approved/recommended dose of JB 14/08/2007 capecitabine in those indications is 1250 mg/m² administered orally twice daily (equivalent to 2500 mg/m² total daily dose) for 14 days followed by a 7-day rest period given as 3-week cycles, for as long as needed. See approved package insert. Typically the mean duration of treatment is 3 to 6 three-week cycles. The currently approved unit dosage forms are a light peach-colored film coated tablet containing 150 mg of capecitabine and a peach-colored film coated tablet containing 500 mg of capecitabine.

The capecitabine tablet currently on the market (Xeloda® Roche) typically requires approximately 7-12 minutes to disintegrate in water (USP Disintegration Test), depending on the size of the tablet. Traditional excipients currently used in these tablets, such as lactose and croscarmellose sodium, by themselves do not overcome the cohesive property of capecitabine in the tablet. The end result is that the marketed tablet slowly disintegrates by surface erosion and is thus not very amenable to rapid dispersion or disintegration in water prior to oral administration to swallowing-compromised patients.

Therefore, the currently marketed capecitabine tablet may be difficult to swallow by the pediatric and geriatric populations, as well as by patients with swallowing impediments and blockages.

A rapidly disintegrating tablet, such as one having a quickly dispersing matrix, and more preferably a rapidly disintegrating flavored tablet, is thus desirable to remedy the foregoing difficulty of slow tablet erosion in water prior to oral administration.

The present invention provides a rapidly disintegrating pharmaceutical dosage form for oral administration of 5'-deoxy-5-fluoro-N-[(pentyloxy)-carbonyl]-cytidine (capecitabine) that is suitable for administration to patients that have difficulty swallowing solid oral dosage forms.

The present invention provides a rapidly disintegrating film coated capecitabine pharmaceutical dosage form suitable for oral administration. Preferably, the tablet disintegrates in water at 37°C (USP Disintegration Test) in less than about 2 minutes, more preferably in less than about 1 minute, and have a hardness of about 8-13 strong Cobb-Units (scu). By manually stirring in water at room temperature, the tablet disintegrates in less than or equal to about 3 minutes. In a preferred embodiment, the composition comprises, based upon the total weight of the final unit dosage form, from about 10% to about 50%, more preferably from about 25% to about 35%, and most preferably 30%, of capecitabine and from about 10% to about 50%, more preferably from about 20% to about 40%, and most preferably 30%, per unit dosage form of at least one disintegrant.

Yet another preferred embodiment of the present invention relates to a lactose free tablet composition for lactose intolerant individuals wherein the lactose is replaced by additional mannitol.

In addition, a directly compressible polyhydric alcohol, such as mannitol, and a microcrystalline cellulose are essential to maintain tablet strength without compromising the disintegration of the tablet. The composition of mannitol comprises from about 2% to about 25%, more preferably from about 4% to about 20% and most preferably 6% to about 16% and the microcrystalline cellulose comprises from about 4% to about 30%, more preferably from about 8% to about 25% and most preferably 12% to about 22% per unit dosage form.

Preferably, the composition comprises from about 50mg to about 1500mg, preferably 100 mg to about 750 mg, and more preferably from about 125 mg to about 500 mg, of capecitabine. Most preferably, the composition comprises, per unit dosage form, 125 mg, 150 mg, 175 mg, 250 mg ,350 mg or 500mg of capecitabine.

Useful disintegrants include, but are not limited to, crospovidone having a particle size in the range of 90% less than 15 microns to a particle size in the range of 90% less than 400 microns, croscarmellose sodium, sodium starch glycolate, low-substituted hydroxypropylcellulose, or any commercially available disintegrant, such as Pharmaburst C™ ,a mannitol/sorbitol combination disclosed and claimed in US Patent No.7,118,765 incorporated herein by reference (available from SPI Pharma, New Castle, Delaware) or any combination thereof.

The pharmaceutical compositions of the invention may include additional therapeutically inert inorganic or organic carriers and excipients. For example, such compositions may include flavorants such as vanillin, bittermasking blend, strawberry flavor or any other flavorant or flavorant combinations which are typically added to pharmaceutical preparations to render them palatable for oral administration.

The compositions may also include sweetening agents such as saccharin sodium, aspartame, sucralose, acesulfame-K, and sucrose.

The compositions may also include binders such as hydroxypropyl methylcellulose, hydroxypropylcellulose, povidone, pregelatinized starch or any other cold swelling corn starch.

The compositions may also include fillers such as lactose anhydrous or microcrystalline cellulose.

The compositions may also include coloring agents, coating agents, antioxidants, stabilizers, lubricants (e.g., magnesium stearate), granulation aids, flow aids, and such other agents and materials as are known to those skilled in the art of making pharmaceutical dosage forms for human oral consumption.

In an embodiment, the unit dosage form is a tablet, preferably a film coated tablet. The coating may contain excipients such as a film former (polymer), a plasticizer, an opacifier, pigments, colorants and the like. The choice of such materials and the amounts to be utilized is considered to be within the art. The film coat composition can be selected from, for example, Hypromellose, Polyvinyl Alcohol, Titanium Dioxide, Talc, Iron Oxide Color without or with plasticizer, such as Polyethylene Glycol, Polysorbate 80, or Triacetin. The following examples illustrate embodiments of unit dosage forms according to the present invention. In each case, the unit dosage form is a film coated tablet.

### Examples 1 -6

### Formulation Composition

| **Examples** | **#1** | **#2** | **#3** | **#4** | **#5** | **#6** |
|---|---|---|---|---|---|---|
| **Ingredients** | **mg/tab** | **mg/tab** | **mg/tab** | **mg/tab** | **mg/tab** | **mg/tab** |
| Capecitabine | 125.00 | 150.00 | 175.00 | 250.00 | 350.00 | 500.00 |
| Lactose Anhydrous | 35.72 | 42.90 | 50.06 | 71.49 | 100.12 | 142.88 |
| Hypromellose | 3.57 | 4.28 | 5.00 | 7.14 | 10.00 | 14.28 |
| Crospovidone | 37.50 | 45.00 | 52.50 | 75.00 | 105.00 | 150.00 |
| Pharmaburst C | 89.30 | 107.16 | 125.00 | 178.60 | 250.00 | 357.20 |
| Mannitol | 23.21 | 27.85 | 32.50 | 46.43 | 65.00 | 92.84 |
| Microcrystalline Cellulose | 46.82 | 56.18 | 65.54 | 93.63 | 131.08 | 187.28 |
| Magnesium Stearate | 8.22 | 9.86 | 11.50 | 16.43 | 23.00 | 32.88 |
| Aspartame | 15.54 | 18.64 | 21.75 | 31.07 | 43.50 | 62.16 |
| Saccharin Sodium | 3.22 | 3.86 | 4.50 | 6.43 | 9.00 | 12.88 |
| Vanillin | 7.86 | 9.43 | 11.00 | 15.71 | 22.00 | 31.44 |
| Bittermasking Blend | 1.47 | 1.76 | 2.06 | 2.94 | 4.12 | 5.88 |
| Strawberry Flavor #915.010 | 2.97 | 3.56 | 4.15 | 5.93 | 8.30 | 11.88 |
| Purified Water¹ | q.s. | q.s. | q.s. | q.s. | q.s. | q.s. |
| Kernel Weight | 400.40 mg | 480.48 mg | 560.56 mg | 800.80 mg | 1121.12 mg | 1601.60 mg |
| Opadry pink film coat | 8.00 | 9.61 | 11.00 | 16.00 | 22.00 | 32.03 |
| Purified Water¹ | 44.53 | 53.44 | 62.34 | 89.05 | 124.67 | 178.10 |
| Total Tablet Weight | 408.40 mg | 490.09 mg | 571.56 mg | 816.80 mg | 1143.12 mg | 1633.63 mg |

| | | | | | | |
|---|---|---|---|---|---|---|
| ¹ removed during processing | | | | | | |

Procedure:
1. Mix Capecitabine with Lactose Anhydrous and a portion of Crospovidone
2. Dissolve Hypromellose in the Purified Water
3. Granulate the blend from Step 1 with the granulating solution from Step 2
4. Wet mill the granulation from Step 3
5. Dry and mill the granules from Step 4
6. Blend the granules from Step 5 with Pharmaburst C, remainder of Crospovidone, Mannitol, Microcrystalline Cellulose, Aspartame, Saccharin Sodium, Vanillin, Bittermasking Blend, and Strawberry Flavor
7. Screen Magnesium Stearate, add it to the blend from Step 6 and mix
8. Compress the tabletting mixture from Step 7 into kernels
9. Prepare the film-coating suspension by dispersing the film coating mixture in the Purified Water
10. Film-coat the kernels from Step 8 using the film-coating suspension from Step 9

### Examples 7-12

The following compositions represent the preferred formulations based on a mg per tablet weight basis whereby Lactose is replaced with Mannitol

### Formulation Composition

| **Examples** | **#7** | **#8** | **#9** | **#10** | **#11** | **#12** |
|---|---|---|---|---|---|---|
| **Ingredients** | **mg/tab** | **mg/tab** | **mg/tab** | **mg/tab** | **mg/tab** | **mg/tab** |
| Capecitabine | 125.00 | 150.00 | 175.00 | 250.00 | 350.00 | 500.00 |
| Mannitol, | 58.93 | 70.75 | 82.56 | 117.92 | 165.12 | 235.72 |
| Hypromellose | 3.57 | 4.28 | 5.00 | 7.14 | 10.00 | 14.38 |
| Crospovidone | 37.50 | 45.00 | 52.50 | 75.00 | 105.00 | 150.00 |
| Pharmaburst C | 89.30 | 107.16 | 125.00 | 178.60 | 250.00 | 357.20 |
| Microcrystalline Cellulose | 46.82 | 56.18 | 65.54 | 93.63 | 131.08 | 187.28 |
| Magnesium Stearate | 8.22 | 9.86 | 11.50 | 16.43 | 23.00 | 32.88 |
| Aspartame | 15.54 | 18.64 | 21.75 | 31.07 | 43.50 | 62.16 |
| Saccharin Sodium | 3.22 | 3.86 | 4.50 | 6.43 | 9.00 | 13.88 |
| Vanillin | 7.86 | 9.43 | 11.00 | 15.71 | 22.00 | 31.44 |
| Bittermasking Blend | 1.47 | 1.76 | 2.06 | 2.94 | 4.12 | 5.88 |
| Strawberry Flavor #915.010 | 2.97 | 3.56 | 4.15 | 5.93 | 8.30 | 11.88 |
| Purified Water | q.s. | q.s. | q.s. | q.s. | q.s. | q.s. |
| Kernel Weight | 400.40 mg | 480.48 mg | 560.56 mg | 800.80 mg | 1121.12 mg | 1601.60 mg |
| Opadry pink film coat | 8.00 | 9.61 | 11.00 | 16.00 | 22.00 | 32.03 |
| Purified Water¹ | 44.53 | 53.44 | 62.34 | 89.05 | 124.67 | 178.10 |
| Total Tablet Weight | 408.40 mg | 490.09 mg | 571.56 mg | 816.80 mg | 1143.12 mg | 1633.63 mg |

| | | | | | | |
|---|---|---|---|---|---|---|
| ¹ removed during processing | | | | | | |

Procedure: Similar to that for Examples 1-6, except replace Lactose Anhydrous with Mannitol in Step 1.

### Examples 13-18

The following compositions represent the preferred formulations based on a mg per tablet weight basis, whereby Pharmaburst C and Lactose are replaced.

### Formulation Composition

| **Examples** | **#13** | **#14** | **#15** | **#16** | **#17** | **#18** |
|---|---|---|---|---|---|---|
| **Ingredients** | **mg/tab** | **mg/tab** | **mg/tab** | **mg/tab** | **mg/tab** | **mg/tab** |
| Capecitabine | 125.00 | 150.00 | 175.00 | 250.00 | 350.00 | 500.00 |
| Mannitol | 58.93 | 70.75 | 82.50 | 117.86 | 165.00 | 235.72 |
| Hypromellose | 3.57 | 4.28 | 5.00 | 7.14 | 10.00 | 14.28 |
| Crospovidone | 62.50 | 75.01 | 87.50 | 125.00 | 175.00 | 250.00 |
| Microcrystalline Cellulose | 82.26 | 98.71 | 115.16 | 164.52 | 230.32 | 329.04 |
| Magnesium Stearate | 7.41 | 8.90 | 10.37 | 14.82 | 20.74 | 29.64 |
| Aspartame | 15.54 | 18.64 | 21.76 | 31.08 | 43.52 | 62.16 |
| Saccharin Sodium | 3.22 | 3.86 | 4.50 | 6.44 | 9.00 | 12.88 |
| Vanillin | 7.86 | 9.43 | 11.00 | 15.72 | 22.00 | 31.44 |
| Bittermasking Blend | 1.47 | 1.76 | 2.06 | 2.94 | 4.12 | 5.88 |
| Strawberry Flavor #915.010 | 2.97 | 3.56 | 4.15 | 5.94 | 8.30 | 11.88 |
| Purified Water¹ | q.s. | q.s. | q.s. | q.s. | q.s. | q.s. |
| Kernel Weight | 370.73 mg | 444.90 mg | 519.00 mg | 741.46 mg | 1038.00 mg | 1482.92 mg |
| Opadry pink film coat | 7.41 | 8.90 | 10.38 | 14.83 | 20.76 | 29.66 |
| Purified Water¹ | 41.99 | 50.43 | 58.82 | 84.04 | 117.64 | 168.07 |
| Total Tablet Weight | 378.14 mg | 453.80 mg | 529.38 mg | 756.29 mg | 1058.76 mg | 1512.58 mg |

| | | | | | | |
|---|---|---|---|---|---|---|
| ¹ removed during processing | | | | | | |

Procedure: Similar to that for Examples 1-6, except replace Lactose Anhydrous with Mannitol in Step 1 and remove Pharmaburst C in Step 6.

### Disintegration Aspects of the Dosage Form

The Following is a comparison of the disintegration times for the rapidly disintegrating tablet of the present invention and the marketed tablet at low and high tablet strengths.

Disintegration times were obtained using the USP Disintegration Apparatus without discs and 37°C Water. The experimental test method and resultant disintegration times observed were performed in accordance with the USP Disintegration Test Method (USP 29, General Chapters, Physical Tests, <709> which is herein incorporated by reference.

For the purposes of this test, disintegration does not imply complete solution of the unit or even of its active constituent. Complete disintegration is defined as that state in which any residue of the unit, except fragments of insoluble coating or capsule shell, remaining on the screen of the test apparatus is a soft mass having no palpably firm core.

### USP DISINTEGRATION APPARATUS

The apparatus consists of a basket-rack assembly, a 1000-mL, low-form beaker, 138 to 160 mm in height and having an inside diameter of 97 to 115 mm for the immersion fluid, a thermostatic arrangement for heating the fluid between 35 and 39 , and a device for raising and lowering the basket in the immersion fluid at a constant frequency rate between 29 and 32 cycles per minute through a distance of not less than 53 mm and not more than 57 mm. The volume of the fluid in the vessel is such that at the highest point of the upward stroke the wire mesh remains at least 15 mm below the surface of the fluid and descends to not less than 25 mm from the bottom of the vessel on the downward stroke. At no time should the top of the basket-rack assembly become submerged. The time required for the upward stroke is equal to the time required for the downward stroke, and the change in stroke direction is a smooth transition, rather than an abrupt reversal of motion. The basket-rack assembly moves vertically along its axis. There is no appreciable horizontal motion or movement of the axis from the vertical.

Basket-Rack Assembly- The basket-rack assembly consists of six open-ended transparent tubes, each 77.5 ± 2.5 mm long and having an inside diameter of 20.7 to 23 mm and a wall 1.0 to 2.8 mm thick; the tubes are held in a vertical position by two plates, each 88 to 92 mm in diameter and 5 to 8.5 mm in thickness, with six holes, each 22 to 26 mm in diameter, equidistant from the center of the plate and equally spaced from one another. Attached to the under surface of the lower plate is a woven stainless steel wire cloth, which has a plain square weave with 1.8- to 2.2-mm apertures and with a wire diameter of 0.57 to 0.66 mm. The parts of the apparatus are assembled and rigidly held by means of three bolts passing through the two plates. A suitable means is provided to suspend the basket-rack assembly from the raising and lowering device using a point on its axis.

The design of the basket-rack assembly may be varied somewhat, provided the specifications for the glass tubes and the screen mesh size are maintained.

Disks- Disks were not used.

### PROCEDURE

Uncoated or Coated Tablets- Place 1 dosage unit in each of the six tubes of the basket. Operate the apparatus using water or the specified medium as the immersion fluid, maintained at 37 ± 2. At the end of the time limit specified in the monograph, lift the basket from the fluid, and observe the tablets to see if all of the tablets have disintegrated completely. If 1 or 2 tablets fail to disintegrate completely, repeat the test on 12 additional tablets. The requirement is met if not fewer than 16 of the total of 18 tablets tested are disintegrated.

**Disintegration Times in Water at 37°C (USP Disintegration Apparatus)**

| **Unit Dosage form** | **Time** |
|---|---|
| Capecitabine - Rapidly Disintegrating Tablets | |
| 125 mg- Example 1 | 50 seconds (0.8 minutes) |
| 500 mg- Example 6 | 55 seconds (0.9 minutes) |
| | |
| 125 mg - Example 7 | seconds (1.2 minutes) |
| 500 mg- Example 12 | seconds (1.8 minutes) |
| | |
| 125 mg - Example 13 | 60 seconds (1 minute) |
| 500 mg - Example 18 | 75 seconds (1.3 minutes) |
| | |
| | |
| Xeloda® Marketed Tablets (available from Roche Laboratories) | |
| 150 mg | 390 seconds (6.5 minutes) |
| 500 mg | 695 seconds (11.6 minutes) |

As demonstrated in the above table, the capecitabine rapidly disintegrating tablets of the invention have disintegration times in water that are approximately eight- to thirteen-fold shorter than the current marketed tablets at their low and high dosage strengths, respectively.

While the invention has been illustrated by reference to specific and preferred embodiments, those skilled in the art will understand that variations and modifications may be made through routine experimentation and practice of the invention. Thus, the invention is intended not to be limited by the foregoing description, but to be defined by the appended claims and their equivalents.

## Claims

1. A film coated pharmaceutical composition comprising capecitabine and at least one disintegrant, said composition disintegrating in water at 37°C in a USP Disintegration Apparatus in less than 2 minutes and having a hardness of about 56-91N (2-13 strong Cobb-Units).

2. The composition of claim 1 in which capecitabine, based upon the total weight of the kernel composition, comprises from about 10% to about 50%.

3. The composition of claim 2 comprising from about 50 mg to about 1500 mg of capecitabine.

4. The composition of claim 3 comprising from about 100 mg to about 750 mg of capecitabine.

5. The composition of claim 3 comprising 125 mg, 175 mg, 250 mg, 350 mg or 500 mg of capecitabine.

6. The pharmaceutical composition of claim 1, wherein at least one disintegrant is selected from the group consisting of crospovidone having a particle size in the range of 90% less than 15 microns to a particle size in the range of 90% less than 400 microns, croscarmellose sodium, sodium starch glycolate, low-substituted hydroxypropylcellulose, Pharmaburst C or any combination of said disintegrants.

7. The composition of claim 5 in which the disintegrant is from about 10 to about 50% per unit dosage form.

8. The composition of claim 6 wherein the disintegrant is from about 20% to about 40% per unit dosage form.

9. The composition of claim 7 wherein the disintegrant is about 30% per unit dosage form.

10. The pharmaceutical composition of claim 1 which contains in addition a directly compressible polyhydric alcohol.

11. The composition of claim 9 wherein the alcohol is mannitol and comprises from about 2% to about 25% per unit dosage form

12. The composition of claim 10 wherein the mannitol comprises about 4% to about 20% per unit dosage form.

13. The composition of claim 11 wherein the mannitol comprises about 6% to about 16% per unit dosage form.

14. The pharmaceutical composition of claim 1 which contains from about 4% to about 30% microcrystalline cellulose per unit dosage form.

15. The composition of claim 13 which contains from about 8% to about 25% microcrystalline cellulose per unit dosage form.

16. The composition of cairn 14 which contains about 12% to about 22% microcrystalline cellulose per unit dosage form.

17. The composition of claim 1 wherein the pharmaceutical composition disintegrates in less than 1 minute.

18. The composition of claim 1 which contains a binder selected from the group consisting of hydroxypropyl methylcellulose, hydroxypropylcellulose, povidone, pregelatinized starch and cold swelling corn starch.

19. The composition of claim 17 wherein capecitabine comprises from about 50 mg to about 1500 mg per unit dosage form.

20. The composition of claim 19 wherein capecitabine comprises from about 100 mg to about 750 mg per unit dosage form.

21. The composition of claim 18 wherein capecitabine comprises 125 mg, 150 mg, 175 mg, 250 mg, 350mg or 500 mg per unit dosage form.

22. A pharmaceutical composition which disintegrates in water at 37°C in a USP Disintegration Apparatus in less than 1 minute comprising capecitabine, at least one disintegrant, a binder, at least one filler, a lubricant, at least one sweetening agent and at least one flavorant.

23. A pharmaceutical composition according to claim 22, **characterized in that** it is film coated.

24. A pharmaceutical composition according to claim 1 or 22, **characterized in that** it is free of lactose.

25. The pharmaceutical composition according to claim 23, comprising 125 mg of Capecitabine, 35.72 mg of Lactose Anhydrous, 3.57 mg of Hypromellose, 37.50 mg of Crospovidone, 89.30 mg of Pharmaburst C, 23.21 mg of Mannitol, 46.82 mg of Microcrystalline Cellulose, 8.22 mg of Magnesium Stearate, 15.54 mg of Aspartame, 3.22 mg of Saccharin Sodium, 7.86 mg of Vanillin, 1.47 mg of Bittermasking Blend and 2.97 mg of Strawberry Flavor.

26. The pharmaceutical composition according to claim 23, comprising 150 mg of Capecitabine, 42.90 mg of Lactose Anhydrous, 4.28 mg of Hypromellose, 45.00 mg of Crospovidone, 107.16 mg of Pharmaburst C, 27.85 mg of Mannitol, 56.18 mg of Microcrystalline Cellulose, 9.86 mg of Magnesium Stearate, 18.64 mg of Aspartame, 3.86 mg of Saccharin Sodium, 9.43 mg of Vanillin, 1.76 mg of Bittermasking Blend and 3.56 mg of Strawberry Flavor.

27. The pharmaceutical composition according to claim 23, comprising 175 mg of Capecitabine, 50.06 mg of Lactose Anhydrous, 5.00 mg of Hypromellose, 52.50 mg of Crospovidone, 125.00 mg of Pharmaburst C, 32.50 mg of Mannitol, 65.54 mg of Microcrystalline Cellulose, 11.50 mg of Magnesium Stearate, 21.75 mg of Aspartame, 4.50 mg of Saccharin Sodium, 11.00 mg of Vanillin, 2.06 mg of Bittermasking Blend and 4.15 mg of Strawberry Flavor.

28. The pharmaceutical composition according to claim 23, comprising 250 mg of Capecitabine, 71.49 mg of Lactose Anhydrous, 7.14 mg of Hypromellose, 75.00 mg of Crospovidone, 178.60 mg of Pharmaburst C, 46.43 mg of Mannitol, 93.63 mg of Microcrystalline Cellulose, 16.43 mg of Magnesium Stearate, 31.07 mg of Aspartame, 6.43 mg of Saccharin Sodium, 15.71 mg of Vanillin, 2.94 mg of Bittermasking Blend and 5.93 mg of Strawberry Flavor.

29. The pharmaceutical composition according to claim 23, comprising 350 mg of Capecitabine, 100.12 mg of Lactose Anhydrous, 10.00 mg of Hypromellose, 105.00 mg of Crospovidone, 250.00 mg of Pharmaburst C, 65.00 mg of Mannitol, 131.08 mg of Microcrystalline Cellulose, 23.00 mg of Magnesium Stearate, 43.50 mg of Aspartame, 9.00 mg of Saccharin Sodium, 22.00 mg of Vanillin, 4.12 mg of Bittermasking Blend and 8.30 mg of Strawberry Flavor.

30. The pharmaceutical composition according to claim 23, comprising 500 mg of Capecitabine, 142.88 mg of Lactose Anhydrous, 14.28 mg of Hypromellose, 150.00 mg of Crospovidone, 357.20 mg of Pharmaburst C, 92.84 mg of Mannitol, 187.28 mg of Microcrystalline Cellulose, 32.88 mg of Magnesium Stearate, 62.16 mg of Aspartame, 12.88 mg of Saccharin Sodium, 31.44 mg of Vanillin, 5.88 mg of Bittermasking Blend and 11.88 mg of Strawberry Flavor.

31. The pharmaceutical composition according to claim 1, comprising 125.00 mg of Capecitabine, 3.57 mg of Hypromellose, 37.50 mg of Crospovidone, 89.30 mg of Pharmaburst C, 58.93 mg of Mannitol, 46.82 mg of Microcrystalline Cellulose, 8.22 mg of Magnesium Stearate, 15.54 mg of Aspartame, 3.22 mg of Saccharin Sodium, 7.86 mg of Vanillin, 1.47 mg of Bittermasking Blend and 2.97 mg of Strawberry Flavor.

32. The pharmaceutical composition according to claim 1, comprising 150.00 mg of Capecitabine, 4.28 mg of Hypromellose, 45.00 mg of Crospovidone, 107.16 mg of Pharmaburst C, 70.75 mg of Mannitol, 56.18 mg of Microcrystalline Cellulose, 9.86 mg of Magnesium Stearate, 18.64 mg of Aspartame, 3.86 mg of Saccharin Sodium, 9.43 mg of Vanillin, 1.76 mg of Bittermasking Blend and 3.56 mg of Strawberry Flavor.

33. The pharmaceutical composition according to claim 1, comprising 175.00 mg of Capecitabine, 5.00 mg of Hypromellose, 52.50 mg of Crospovidone, 125.00 mg of Pharmaburst C, 82.56 mg of Mannitol, 65.54 mg of Microcrystalline Cellulose, 11.50 mg of Magnesium Stearate, 21.75 mg of Aspartame, 4.50 mg of Saccharin Sodium, 11.00 mg of Vanillin, 2.06 mg of Bittermasking Blend and 4.15 mg of Strawberry Flavor.

34. The pharmaceutical composition according to claim 1, comprising 250.00 mg of Capecitabine, 7.14 mg of Hypromellose, 75.00 mg of Crospovidone, 178.60 mg of Pharmaburst C, 117.92 mg of Mannitol, 93.63 mg of Microcrystalline Cellulose, 16.43 mg of Magnesium Stearate, 31.07 mg of Aspartame, 6.43 mg of Saccharin Sodium, 15.71 mg of Vanillin, 2.94 mg of Bittermasking Blend and 5.93 mg of Strawberry Flavor.

35. The pharmaceutical composition according to claim 1, comprising 350.00 mg of Capecitabine, 10.00 mg of Hypromellose, 105.00 mg of Crospovidone, 250.00 mg of Pharmaburst C, 165.12 mg of Mannitol, 131.08 mg of Microcrystalline Cellulose, 23.00 mg of Magnesium Stearate, 43.50 mg of Aspartame, 9.00 mg of Saccharin Sodium, 22.00 mg of Vanillin, 4.12 mg of Bittermasking Blend and 8.30 mg of Strawberry Flavor.

36. The pharmaceutical composition according to claim 1, comprising 500.00 mg of Capecitabine, 14.28 mg of Hypromellose, 150.00 mg of Crospovidone, 357.20 mg of Pharmaburst C, 235.72 mg of Mannitol, 187.28 mg of Microcrystalline Cellulose, 32.88 mg of Magnesium Stearate, 62.16 mg of Aspartame, 12.88 mg of Saccharin Sodium, 31.44 mg of Vanillin, 5.88 mg of Bittermasking Blend and 11.88 mg of Strawberry Flavor.

37. The pharmaceutical composition according to claim 1, comprising 125.00 mg of Capecitabine, 3.57 mg of Hypromellose, 62.50 mg of Crospovidone, 58.93 mg of Mannitol, 82.26 mg of Microcrystalline Cellulose, 7.41 mg of Magnesium Stearate, 15.54 mg of Aspartame, 3.22 mg of Saccharin Sodium, 7.86 mg of Vanillin, 1.47 mg of Bittermasking Blend and 2.97 mg of Strawberry Flavor.

38. The pharmaceutical composition according to claim 1, comprising 150.00 mg of Capecitabine, 4.28 mg of Hypromellose, 75.01 mg of Crospovidone, 70.75 mg of Mannitol, 98.71 mg of Microcrystalline Cellulose, 8.90 mg of Magnesium Stearate, 18.64 mg of Aspartame, 3.86 mg of Saccharin Sodium, 9.43 mg of Vanillin, 1.76 mg of Bittermasking Blend and 3.56 mg of Strawberry Flavor.

39. The pharmaceutical composition according to claim 1, comprising 175.00 mg of Capecitabine, 5.00 mg of Hypromellose, 87.50 mg of Crospovidone, 82.50 mg of Mannitol, 115.16 mg of Microcrystalline Cellulose, 10.37 mg of Magnesium Stearate, 21.76 mag of Aspartame, 4.50 mg of Saccharin Sodium, 11.00 mg of Vanillin, 2.06 mg of Bittermasking Blend and 4.15 mg of Strawberry Flavor.

40. The pharmaceutical composition according to claim 1, comprising 250.00 mg of Capecitabine, 7.14 mg of Hypromellose, 125.00 mg of Crospovidone, 117.86 mg of Mannitol, 164.52 mg of Microcrystalline Cellulose, 14.82 mg of Magnesium Stearate, 31.08 mg of Aspartame, 6.44 mg of Saccharin Sodium, 15.72 mg of Vanillin, 2.94 mg of Bittermasking Blend and 5.94 mg of Strawberry Flavor.

41. The pharmaceutical composition according to claim 1, comprising 350.00 mg of Capecitabine, 10.00 mg of Hypromellose, 175.00 mg of Crospovidone, 165.00 mg of Mannitol, 230.32 mg of Microcrystalline Cellulose, 20.74 mg of Magnesium Stearate, 43.52 mg of Aspartame, 9.00 mg of Saccharin Sodium, 22.00 mg of Vanillin, 4.12 mg of Bittermasking Blend and 8.30 mg of Strawberry Flavor.

42. The pharmaceutical composition according to claim 1, comprising 500.00 mg of Capecitabine, 14.28 mg of Hypromellose, 250.00 mg of Crospovidone, 235.72 mg of Mannitol, 329.04 mg of Microcrystalline Cellulose, 29.64 mg of Magnesium Stearate, 62.16 mg of Aspartame, 12.88 mg of Saccharin Sodium, 31.44 mg of Vanillin, 5.88 mg of Bittermasking Blend and 11.88 mg of Strawberry Flavor.

## Patentansprüche

1. Filmbeschichtete pharmazeutische Zusammensetzung umfassend Capecitabin und mindestens ein Sprengmittel, wobei sich die Zusammensetzung in Wasser bei 37°C in einem USP-Auflösungsgerät in weniger als 2 Minuten auflöst und eine Härte von etwa 56-91 N (8-13 starke Cobb-Einheiten) besitzt.

2. Zusammensetzung nach Anspruch 1, in der Capecitabin, ausgehend vom Gesamtgewicht der Kemzusammensetzung, von etwa 10% bis etwa 50% umfasst.

3. Zusammensetzung nach Anspruch 2, umfassend von etwa 50 mg bis etwa 1500 mg Capecitabin.

4. Zusammensetzung nach Anspruch 3, umfassend von etwa 100 mg bis etwa 750 mg Capecitabin.

5. Zusammensetzung nach Anspruch 3, umfassend 125 mg, 175 mg, 250 mg, 350 mg oder 500 mg Capecitabin.

6. Pharmazeutische Zusammensetzung nach Anspruch 1, wobei mindestens ein Sprengmittel ausgewählt ist aus der Gruppe bestehend aus Crospovidon, das eine Partikelgröße im Bereich von 90% weniger als 15 Mikrometer bis zu einer Partikelgröße im Bereich von 90% weniger als 400 Mikrometer besitzt, Croscarmellose-Natrium, Natrium-Stärke-Glykolat, niedrig substituierter Hydroxypropylcellulose, Pharmaburst C oder einer beliebigen Kombination dieser Sprengmittel.

7. Zusammensetzung nach Anspruch 5, in der das Sprengmittel von etwa 10 bis etwa 50% pro Einheitsdosierungsform vorliegt.

8. Zusammensetzung nach Anspruch 6, wobei das Sprengmittel von etwa 20% bis etwa 40% pro Einheitsdosierungsform vorliegt.

9. Zusammensetzung nach Anspruch 7, wobei das Sprengmittel zu etwa 30% pro Einheitsdosierungsform vorliegt.

10. Pharmazeutische Zusammensetzung nach Anspruch 1, die zusätzlich einen direkt verpressbaren mehrwertigen Alkohol enthält.

11. Zusammensetzung nach Anspruch 9, wobei der Alkohol Mannitol ist und von etwa 2% bis etwa 25% pro Einheitsdosierungsform umfasst.

12. Zusammensetzung nach Anspruch 10, wobei das Mannitol etwa 4% bis etwa 20% pro Einheitsdosierungsform umfasst.

13. Zusammensetzung nach Anspruch 11, wobei das Mannitol etwa 6% bis etwa 16% pro Einheitsdosierungsform umfasst.

14. Pharmazeutische Zusammensetzung nach Anspruch 1, die von etwa 4% bis etwa 30% mikrokristalline Cellulose pro Einheitsdosierungsform enthält.

15. Zusammensetzung nach Anspruch 13, die von etwa 8% bis etwa 25% mikrokristalline Cellulose pro Einheitsdosierungsform enthält.

16. Zusammensetzung nach Anspruch 14, die etwa 12% bis etwa 22% mikrokristalline Cellulose pro Einheitsdosierungsform enthält.

17. Zusammensetzung nach Anspruch 1, wobei sich die pharmazeutische Zusammensetzung in weniger als 1 Minute auflöst.

18. Zusammensetzung nach Anspruch 1, die ein Bindemittel ausgewählt aus der Gruppe bestehend aus Hydroxypropylmethylcellulose, Hydroxypropylcellulose, Povidon, vorgelatinierter Stärke und kaltquellender Maisstärke enthält.

19. Zusammensetzung nach Anspruch 17, wobei Capecitabin von etwa 50 mg bis etwa 1500 mg pro Einheitsdosierungsform umfasst.

20. Zusammensetzung nach Anspruch 19, wobei Capecitabin von etwa 100 mg bis etwa 750 mg pro Einheitsdosierungsform umfasst.

21. Zusammensetzung nach Anspruch 18, wobei Capecitabin 125 mg, 150 mg, 175 mg, 250 mg, 350 mg oder 500 mg pro Einheitsdosierungsform umfasst.

22. Pharmazeutische Zusammensetzung, die sich in Wasser bei 37°C in einem USP-Auflösungsgerät in weniger als 1 Minute auflöst, umfassend Capecitabin, mindestens ein Sprengmittel, ein Bindemittel, mindestens einen Füllstoff, ein Gleitmittel, mindestens ein Süßungsmittel und mindestens einen Geschmacksstoff.

23. Pharmazeutische Zusammensetzung nach Anspruch 22, **dadurch gekennzeichnet, dass** sie filmbeschichtet ist.

24. Pharmazeutische Zusammensetzung nach Anspruch 1 oder 22, **dadurch gekennzeichnet, dass** sie frei von Lactose ist.

25. Pharmazeutische Zusammensetzung nach Anspruch 23, umfassend 125 mg Capecitabin, 35,72 mg wasserfreie Lactose, 3,57 mg Hypromellose, 37,50 mg Crospovidon, 89,30 mg Pharmaburst C, 23,21 mg Mannitol, 46,82 mg mikrokristalline Cellulose, 8,22 mg Magnesiumstearat, 15,54 mg Aspartam, 3,22 mg Saccharin-Natrium, 7,86 mg Vanillin, 1,47 mg Mischung zur Maskierung von Bitterstoffen und 2,97 mg Erdbeergeschmack.

26. Pharmazeutische Zusammensetzung nach Anspruch 23, umfassend 150 mg Capecitabin, 42,90 mg wasserfreie Lactose, 4,28 mg Hypromellose, 45,00 mg Crospovidon, 107,16 mg Pharmaburst C, 27,85 mg Mannitol, 56,18 mg mikrokristalline Cellulose, 9,86 mg Magnesiumstearat, 18,64 mg Aspartam, 3,86 mg Saccharin-Natrium, 9,43 mg Vanillin, 1,76 mg Mischung zur Maskierung von Bitterstoffen und 3,56 mg Erdbeergeschmack.

27. Pharmazeutische Zusammensetzung nach Anspruch 23, umfassend 175 mg Capecitabin, 50,06 mg wasserfrei Lactose, 5,00 mg Hypromellose, 52,50 mg Crospovidon, 125,00 mg Pharmaburst C, 32,50 mg Mannitol, 65,54 mg mikrokristalline Cellulose, 11,50 mg Magnesiumstearat, 21,75 mg Aspartam, 4,50 mg Saccharin-Natrium, 11,00 mg Vanillin, 2,06 mg Mischung zur Maskierung von Bitterstoffen und 4,15 mg Erdbeergeschmack.

28. Pharmazeutische Zusammensetzung nach Anspruch 23, umfassend 250 mg Capecitabin, 71,49 mg wasserfreie Lactose, 7,14 mg Hypromellose, 75,00 mg Crospovidon, 178,60 mg Pharmaburst C, 46,43 mg Mannitol, 93,63 mg mikrokristalline Cellulose, 16,43 mg Magnesiumstearat, 31,07 mg Aspartam, 6,43 mg Saccharin-Natrium, 15,71 mg Vanillin, 2,94 mg Mischung zur Maskierung von Bitterstoffen und 5,93 mg Erdbeergeschmack.

29. Pharmazeutische Zusammensetzung nach Anspruch 23, umfassend 350 mg Capecitabin, 100,12 mg wasserfreie Lactose, 10,00 mg Hypromellose, 105,00 mg Crospovidon, 250,00 mg Pharmaburst C, 65,00 mg Mannitol, 131,08 mg mikrokristalline Cellulose, 23,00 mg Magnesiumstearat, 43,50 mg Aspartam, 9,00 mg Saccharin-Natrium, 22,00 mg Vanillin, 4,12 mg Mischung zur Maskierung von Bitterstoffen und 8,30 mg Erdbeergeschmack.

30. Pharmazeutische Zusammensetzung nach Anspruch 23, umfassend 500 mg Capecitabin, 142,88 mg wasserfreie Lactose, 14,28 mg Hypromellose, 150,00 mg Crospovidon, 357,20 mg Pharmaburst C, 92,84 mg Mannitol, 187,28 mg mikrokristalline Cellulose, 32,88 mg Magnesiumstearat, 62,16 mg Aspartam, 12,88 mg Saccharin-Natrium, 31,44 mg Vanillin, 5,88 mg Mischung zur Maskierung von Bitterstoffen und 11,88 mg Erdbeergeschmack.

31. Pharmazeutische Zusammensetzung nach Anspruch 1, umfassend 125,00 mg Capecitabin, 3,57 mg Hypromellose, 37,50 mg Crospovidon, 89,30 mg Pharmaburst C, 58,93 mg Mannitol, 46,82 mg mikrokristalline Cellulose, 8,22 mg Magnesiumstearat, 15,54 mg Aspartam, 3,22 mg Saccharin-Natrium, 7,86 mg Vanillin, 1,47 mg Mischung zur Maskierung von Bitterstoffen und 2,97 mg Erdbeergeschmack.

32. Pharmazeutische Zusammensetzung nach Anspruch 1, umfassend 150,00 mg Capecitabin, 4,28 mg Hypromellose, 45,00 mg Crospovidon, 107,16 mg Pharmaburst C, 70,75 mg Mannitol, 56,18 mg mikrokristalline Cellulose, 9,86 mg Magnesiumstearat, 18,64 mg Aspartam, 3,86 mg Saccharin-Natrium, 9,43 mg Vanillin, 1,76 mg Mischung zur Maskierung von Bitterstoffen und 3,56 mg Erdbeergeschmack.

33. Pharmazeutische Zusammensetzung nach Anspruch 1, umfassend 175,00 mg Capecitabin, 5,00 mg Hypromellose, 52,50 mg Crospovidon, 125,00 mg Pharmaburst C, 82,56 mg Mannitol, 65,54 mg mikrokristalline Cellulose, 11,50 mg Magnesiumstearat, 21,75 mg Aspartam, 4,50 mg Saccharin-Natrium, 11,00 mg Vanillin, 2,06 mg Mischung zur Maskierung von Bitterstoffen und 4,15 mg Erdbeergeschmack.

34. Pharmazeutische Zusammensetzung nach Anspruch 1, umfassend 250,00 mg Capecitabin, 7,14 mg Hypromellose, 75,00 mg Crospovidon, 178,60 mg Pharmaburst C, 117,92 mg Mannitol, 93,63 mg mikrokristalline Cellulose, 16,43 mg Magnesiumstearat, 31,07 mg Aspartam, 6,43 mg Saccharin-Natrium, 15,71 mg Vanillin, 2,94 mg Mischung zur Maskierung von Bitterstoffen und 5,93 mg Erdbeergeschmack.

35. Pharmazeutische Zusammensetzung nach Anspruch 1, umfassend 350,00 mg Capecitabin, 10,00 mg Hypromellose, 105,00 mg Crospovidon, 250,00 mg Pharmaburst C, 165,12 mg Mannitol, 131,08 mg mikrokristalline Cellulose, 23,00 mg Magnesiumstearat, 43,50 mg Aspartam, 9,00 mg Saccharin-Natrium, 22,00 mg Vanillin, 4,12 mg Mischung zur Maskierung von Bitterstoffen und 8,30 mg Erdbeergeschmack.

36. Pharmazeutische Zusammensetzung nach Anspruch 1, umfassend 500,00 mg Capecitabin, 14,28 mg Hypromellose, 150,00 mg Crospovidon, 357,20 mg Pharmaburst C, 235,72 mg Mannitol, 187,28 mg mikrokristalline Cellulose, 32,88 mg Magnesiumstearat, 62,16 mg Aspartam, 12,88 mg Saccharin-Natrium, 31,44 mg Vanillin, 5,88 mg Mischung zur Maskierung von Bitterstoffen und 11,88 mg Erdbeergeschmack.

37. Pharmazeutische Zusammensetzung nach Anspruch 1, umfassend 125,00 mg Capecitabin, 3,57 mg Hypromellose, 62,50 mg Crospovidon, 58,93 mg Mannitol, 82,26 mg mikrokristalline Cellulose, 7,41 mg Magnesiumstearat, 15,54 mg Aspartam, 3,22 mg Saccharin-Natrium, 7,86 mg Vanillin, 1,47 mg Mischung zur Maskierung von Bitterstoffen und 2,97 mg Erdbeergeschmack.

38. Pharmazeutische Zusammensetzung nach Anspruch 1, umfassend 150,00 mg Capecitabin, 4,28 mg Hypromellose, 75,01 mg Crospovidon, 70,75 mg Mannitol, 98,71 mg mikrokristalline Cellulose, 8,90 mg Magnesiumstearat, 18,64 mg Aspartam, 3,86 mg Saccharin-Natrium, 9,43 mg Vanillin, 1,76 mg Mischung zur Maskierung von Bitterstoffen und 3,56 mg Erdbeergeschmack.

39. Pharmazeutische Zusammensetzung nach Anspruch 1, umfassend 175,00 mg Capecitabin, 5,00 mg Hypromellose, 87,50 mg Crospovidon, 82,50 mg Mannitol, 115,16 mg mikrokristalline Cellulose, 10,37 mg Magnesiumstearat, 21,76 mg Aspartam, 4,50 mg Saccharin-Natrium, 11,00 mg Vanillin, 2,06 mg Mischung zur Maskierung von Bitterstoffen und 4,15 mg Erdbeergeschmack.

40. Pharmazeutische Zusammensetzung nach Anspruch 1, umfassend 250,00 mg Capecitabin, 7,14 mg Hypromellose, 125,00 mg Crospovidon, 117,86 mg Mannitol, 164,52 mg mikrokristalline Cellulose, 14,82 mg Magnesiumstearat, 31,08 mg Aspartam, 6,44 mg Saccharin-Natrium, 15,72 mg Vanillin, 2,94 mg Mischung zur Maskierung von Bitterstoffen und 5,94 mg Erdbeergeschmack.

41. Pharmazeutische Zusammensetzung nach Anspruch 1, umfassend 350,00 mg Capecitabin, 10,00 mg Hypromellose, 175,00 mg Crospovidon, 165,00 mg Mannitol, 230,32 mg mikrokristalline Cellulose, 20,74 mg Magnesiumstearat, 43,52 mg Aspartam, 9,00 mg Saccharin-Natrium, 22,00 mg Vanillin, 4,12 mg Mischung zur Maskierung von Bitterstoffen und 8,30 mg Erdbeergeschmack.

42. Pharmazeutische Zusammensetzung nach Anspruch 1, umfassend 500,00 mg Capecitabin, 14,28 mg Hypromellose, 250,00 mg Crospovidon, 235,72 mg Mannitol, 329,04 mg mikrokristalline Cellulose, 29,64 mg Magnesiumstearat, 62,16 mg Aspartam, 12,88 mg Saccharin-Natrium, 31,44 mg Vanillin, 5,88 mg Mischung zur Maskierung von Bitterstoffen und 11,88 mg Erdbeergeschmack.

## Revendications

1. Composition pharmaceutique pelliculée, comprenant de la capécitabine et au moins un désintégrant, ladite composition se désintégrant dans l'eau à 37°C dans un Appareil de Désintégration USP en moins de 2 minutes et ayant une dureté d'environ 56-91 N (8-13 unités Strong-Cobb).

2. Composition selon la revendication 1, dans laquelle la capécitabine représente environ 10 % à environ 50 % de la masse totale de la composition du noyau.

3. Composition selon la revendication 2, comprenant d'environ 50 mg à environ 1500 mg de capécitabine.

4. Composition selon la revendication 3, comprenant d'environ 100 mg à environ 750 mg de capécitabine.

5. Composition selon la revendication 3, comprenant 125 mg, 175 mg, 250 mg, 350 mg ou 500 mg de capécitabine.

6. Composition pharmaceutique selon la revendication 1, dans laquelle au moins un désintégrant est choisi dans le groupe constitué de la crospovidone ayant une taille de particules à 90 % dans le domaine inférieur à 15 microns à une taille de particules à 90 % dans le domaine inférieur à 400 microns, la croscarmellose sodium, le glycolate de sodium et d'amidon, l'hydroxypropylcellulose à faible degré de substitution, le Pharmaburst C ou une combinaison quelconque desdits désintégrants.

7. Composition selon la revendication 5, dans laquelle le désintégrant représente environ 10 à environ 50 % d'une forme galénique unitaire.

8. Composition selon la revendication 6, dans laquelle le désintégrant représente environ 20 à environ 40 % d'une forme galénique unitaire.

9. Composition selon la revendication 7, dans laquelle le désintégrant représente environ 30 % d'une forme galénique unitaire.

10. Composition pharmaceutique selon la revendication 1, qui contient en outre un alcool polyhydrique pouvant être comprimé directement.

11. Composition selon la revendication 9, dans laquelle l'alcool est le mannitol et représente environ 2 % à environ 25 % d'une forme galénique unitaire.

12. Composition selon la revendication 10, dans laquelle le mannitol représente environ 4 % à environ 20 % d'une forme galénique unitaire.

13. Composition selon la revendication 11, dans laquelle le mannitol représente environ 6 % à environ 16 % d'une forme galénique unitaire.

14. Composition pharmaceutique selon la revendication 1, qui contient d'environ 4 % à environ 30 % de cellulose microcristalline par forme galénique unitaire.

15. Composition selon la revendication 13, qui contient d'environ 8 % à environ 25 % de cellulose microcristalline par forme galénique unitaire.

16. Composition selon la revendication 14, qui contient d'environ 12 % à environ 22 % de cellulose microcristalline par forme galénique unitaire.

17. Composition selon la revendication 1, la composition pharmaceutique se désintégrant en moins de 1 minute.

18. Composition selon la revendication 1, qui contient un liant choisi dans le groupe constitué de l'hydroxypropylméthylcellulose, l'hydroxypropylcellulose, la povidone, l'amidon prégélatinisé et l'amidon de maïs gonflant à froid.

19. Composition selon la revendication 17, comprenant d'environ 50 mg à environ 1500 mg de capécitabine par forme galénique unitaire.

20. Composition selon la revendication 19, comprenant d'environ 100 mg à environ 750 mg de capécitabine par forme galénique unitaire.

21. Composition selon la revendication 18, comprenant 125 mg, 150 mg, 175 mg, 250 mg, 350 mg ou 500 mg de capécitabine par forme galénique unitaire.

22. Composition pharmaceutique qui se désintègre dans l'eau à 37°C dans un Appareil de Désintégration USP en moins d'une minute, comprenant de la capécitabine, au moins un désintégrant, un liant, au moins une charge, un lubrifiant, au moins un édulcorant et au moins un aromatisant.

23. Composition pharmaceutique selon la revendication 22, **caractérisée en ce qu'**elle est pelliculée.

24. Composition pharmaceutique selon la revendication 1 ou la revendication 22, **caractérisée en ce qu'**elle ne contient pas de lactose.

25. Composition pharmaceutique selon la revendication 23, comprenant 125 mg de capécitabine, 35,72 mg de lactose anhydre, 3,57 mg d'Hypromellose, 37,50 mg de Crospovidone, 89,30 mg de Pharmaburst C, 23,21 mg de mannitol, 46,82 mg de cellulose microcristalline, 8,22 mg de stéarate de magnésium, 15,54 mg d'aspartame, 3,22 mg de saccharine sodique, 7,86 mg de vanilline, 1,47 mg de mélange masquant l'amertume et 2,97 mg d'arôme de fraise.

26. Composition pharmaceutique selon la revendication 23, comprenant 150 mg de capécitabine, 42,90 mg de lactose anhydre, 4,28 mg d'Hypromellose, 45,00 mg de Crospovidone, 107,16 mg de Pharmaburst C, 27,85 mg de mannitol, 56,18 mg de cellulose microcristalline, 9,86 mg de stéarate de magnésium, 18,64 mg d'aspartame, 3,86 mg de saccharine sodique, 9,43 mg de vanilline, 1,76 mg de mélange masquant l'amertume et 3,56 mg d'arôme de fraise.

27. Composition pharmaceutique selon la revendication 23, comprenant 175 mg de capécitabine, 50,06 mg de lactose anhydre, 5,00 mg d'Hypromellose, 52,50 mg de Crospovidone, 125,00 mg de Pharmaburst C, 32,50 mg de mannitol, 65,54 mg de cellulose microcristalline, 11,50 mg de stéarate de magnésium, 21,75 mg d'aspartame, 4,50 mg de saccharine sodique, 11,00 mg de vanilline, 2,06 mg de mélange masquant l'amertume et 4,15 mg d'arôme de fraise.

28. Composition pharmaceutique selon la revendication 23, comprenant 250 mg de capécitabine, 71,49 mg de lactose anhydre, 7,14 mg d'Hypromellose, 75,00 mg de Crospovidone, 178,60 mg de Pharmaburst C, 46,43 mg de mannitol, 93,63 mg de cellulose microcristalline, 16,43 mg de stéarate de magnésium, 31,07 mg d'aspartame, 6,43 mg de saccharine sodique, 15,71 mg de vanilline, 2,94 mg de mélange masquant l'amertume et 5,93 mg d'arôme de fraise.

29. Composition pharmaceutique selon la revendication 23, comprenant 350 mg de capécitabine, 100,12 mg de lactose anhydre, 10,00 mg d'Hypromellose, 105,00 mg de Crospovidone, 250,00 mg de Pharmaburst C, 65,00 mg de mannitol, 131,08 mg de cellulose microcristalline, 23,00 mg de stéarate de magnésium, 43,50 mg d'aspartame, 9,00 mg de saccharine sodique, 22,00 mg de vanilline, 4,12 mg de mélange masquant l'amertume et 8,30 mg d'arôme de fraise.

30. Composition pharmaceutique selon la revendication 23, comprenant 500 mg de capécitabine, 142,88 mg de lactose anhydre, 14,28 mg d'Hypromellose, 150,00 mg de Crospovidone, 357,20 mg de Pharmaburst C, 92,84 mg de mannitol, 187,28 mg de cellulose microcristalline, 32,88 mg de stéarate de magnésium, 62,16 mg d'aspartame, 12,88 mg de saccharine sodique, 31,44 mg de vanilline, 5,88 mg de mélange masquant l'amertume et 11,88 mg d'arôme de fraise.

31. Composition pharmaceutique selon la revendication 1, comprenant 125,00 mg de capécitabine, 3,57 mg d'Hypromellose, 37,50 mg de Crospovidone, 89,30 mg de Pharmaburst C, 58,93 mg de mannitol, 46,82 mg de cellulose microcristalline, 8,22 mg de stéarate de magnésium, 15,54 mg d'aspartame, 3,22 mg de saccharine sodique, 7,86 mg de vanilline, 1,47 mg de mélange masquant l'amertume et 2,97 mg d'arôme de fraise.

32. Composition pharmaceutique selon la revendication 1, comprenant 150,00 mg de capécitabine, 4,28 mg d'Hypromellose, 45,00 mg de Crospovidone, 107,16 mg de Pharmaburst C, 70,75 mg de mannitol, 56,18 mg de cellulose microcristalline, 9,86 mg de stéarate de magnésium, 18,64 mg d'aspartame, 3,86 mg de saccharine sodique, 9,43 mg de vanilline, 1,76 mg de mélange masquant l'amertume et 3,56 mg d'arôme de fraise.

33. Composition pharmaceutique selon la revendication 1, comprenant 175,00 mg de capécitabine, 5,00 mg d'Hypromellose, 52,50 mg de Crospovidone, 125,00 mg de Pharmaburst C, 82,56 mg de mannitol, 65,54 mg de cellulose microcristalline, 11,50 mg de stéarate de magnésium, 21,75 mg d'aspartame, 4,50 mg de saccharine sodique, 11,00 mg de vanilline, 2,06 mg de mélange masquant l'amertume et 4,15 mg d'arôme de fraise.

34. Composition pharmaceutique selon la revendication 1, comprenant 250,00 mg de capécitabine, 7,14 mg d'Hypromellose, 75,00 mg de Crospovidone, 178,60 mg de Pharmaburst C, 117,92 mg de mannitol, 93,63 mg de cellulose microcristalline, 16,43 mg de stéarate de magnésium, 31,07 mg d'aspartame, 6,43 mg de saccharine sodique, 15,71 mg de vanilline, 2,94 mg de mélange masquant l'amertume et 5,93 mg d'arôme de fraise.

35. Composition pharmaceutique selon la revendication 1, comprenant 350,00 mg de capécitabine, 10,00 mg d'Hypromellose, 105,00 mg de Crospovidone, 250,00 mg de Pharmaburst C, 165,12 mg de mannitol, 131,08 mg de cellulose microcristalline, 23,00 mg de stéarate de magnésium, 43,50 mg d'aspartame, 9,00 mg de saccharine sodique, 22,00 mg de vanilline, 4,12 mg de mélange masquant l'amertume et 8,30 mg d'arôme de fraise.

36. Composition pharmaceutique selon la revendication 1, comprenant 500,00 mg de capécitabine, 14,28 mg d'Hypromellose, 150,00 mg de Crospovidone, 357,20 mg de Pharmaburst C, 235,72 mg de mannitol, 187,28 mg de cellulose microcristalline, 32,88 mg de stéarate de magnésium, 62,16 mg d'aspartame, 12,88 mg de saccharine sodique, 31,44 mg de vanilline, 5,88 mg de mélange masquant l'amertume et 11,88 mg d'arôme de fraise.

37. Composition pharmaceutique selon la revendication 1, comprenant 125,00 mg de capécitabine, 3,57 mg d'Hypromellose, 62,50 mg de Crospovidone, 58,93 mg de mannitol, 82,26 mg de cellulose microcristalline, 7,41 mg de stéarate de magnésium, 15,54 mg d'aspartame, 3,22 mg de saccharine sodique, 7,86 mg de vanilline, 1,47 mg de mélange masquant l'amertume et 2,97 mg d'arôme de fraise.

38. Composition pharmaceutique selon la revendication 1, comprenant 150,00 mg de capécitabine, 4,28 mg d'Hypromellose, 75,01 mg de Crospovidone, 70,75 mg de mannitol, 98,71 mg de cellulose microcristalline, 8,90 mg de stéarate de magnésium, 18,64 mg d'aspartame, 3,86 mg de saccharine sodique, 9,43 mg de vanilline, 1,76 mg de mélange masquant l'amertume et 3,56 mg d'arôme de fraise.

39. Composition pharmaceutique selon la revendication 1, comprenant 175,00 mg de capécitabine, 5,00 mg d'Hypromellose, 87,50 mg de Crospovidone, 82,50 mg de mannitol, 115,16 mg de cellulose microcristalline, 10,37 mg de stéarate de magnésium, 21,76 mg d'aspartame, 4,50 mg de saccharine sodique, 11,00 mg de vanilline, 2,06 mg de mélange masquant l'amertume et 4,15 mg d'arôme de fraise.

40. Composition pharmaceutique selon la revendication 1, comprenant 250,00 mg de capécitabine, 7,14 mg d'Hypromellose, 125,00 mg de Crospovidone, 117,86 mg de mannitol, 164,52 mg de cellulose microcristalline, 14,82 mg de stéarate de magnésium, 31,08 mg d'aspartame, 6,44 mg de saccharine sodique, 15,72 mg de vanilline, 2,94 mg de mélange masquant l'amertume et 5,94 mg d'arôme de fraise.

41. Composition pharmaceutique selon la revendication 1, comprenant 350,00 mg de capécitabine, 10,00 mg d'Hypromellose, 175,00 mg de Crospovidone, 165,00 mg de mannitol, 230,32 mg de cellulose microcristalline, 20,74 mg de stéarate de magnésium, 43,52 mg d'aspartame, 9,00 mg de saccharine sodique, 22,00 mg de vanilline, 4,12 mg de mélange masquant l'amertume et 8,30 mg d'arôme de fraise.

42. Composition pharmaceutique selon la revendication 1, comprenant 500,00 mg de capécitabine, 14,28 mg d'Hypromellose, 250,00 mg de Crospovidone, 235,72 mg de mannitol, 329,04 mg de cellulose microcristalline, 29,64 mg de stéarate de magnésium, 62,16 mg d'aspartame, 12,88 mg de saccharine sodique, 31,44 mg de vanilline, 5,88 mg de mélange masquant l'amertume et 11,88 mg d'arôme de fraise.
